(19) Europäisches Patentamt European Patent Office Office européen des brevets

(11) **EP 4 378 439 A1**

(12) **EUROPEAN PATENT APPLICATION**
published in accordance with Art. 153(4) EPC

(43) Date of publication:
**05.06.2024 Bulletin 2024/23**

(21) Application number: **22863974.6**

(22) Date of filing: **06.06.2022**

(51) International Patent Classification (IPC):
**A61F 13/49** *(2006.01)*    **A61F 13/514** *(2006.01)*

(52) Cooperative Patent Classification (CPC):
**A61F 13/49; A61F 13/514**

(86) International application number:
**PCT/JP2022/022837**

(87) International publication number:
**WO 2023/032386 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2021 JP 2021141306**

(71) Applicant: **Unicharm Corporation**
**Ehime 799-0111 (JP)**

(72) Inventors:
• **NAITO Shimpei**
**Kanonji-shi, Kagawa 769-1602 (JP)**
• **KURITA Noriyuki**
**Kanonji-shi, Kagawa 769-1602 (JP)**

(74) Representative: **karo IP**
**karo IP Patentanwälte**
**Kahlhöfer Rößler Kreuels PartG mbB**
**Postfach 32 01 02**
**40416 Düsseldorf (DE)**

(54) **UNDERWEAR-TYPE ABSORBENT ARTICLE**

(57) An underwear-type absorbent article including: an absorbent body including a liquid-absorbent absorbent core; a belt part including a ventral waistline part located on one end side in a longitudinal direction of the absorbent body and a dorsal waistline part located on another end side in the longitudinal direction of the absorbent body; and a plurality of crotch elastic members disposed on the absorbent core and extending in the longitudinal direction, in which at least one or more of the crotch elastic members are disposed in each of three regions divided equally in a width direction intersecting the longitudinal direction in the absorbent core.

**Fig.1**

EP 4 378 439 A1

## Description

## Technical Field

One aspect of the present invention relates to an underwear-type absorbent article.

## Background Art

Patent Literature 1 describes an underwear-type absorbent article including: a tubular waistline part including a ventral side exterior sheet covering a ventral side of a waistline of a wearer and a dorsal side exterior sheet covering a dorsal side, the tubular waistline part being formed by joining a joint part at both side edges in a width direction of the ventral side exterior sheet and a joint part at both side edges in the width direction of the dorsal side exterior sheet; and an absorbent body including a front end connected to an inner surface of a central part in the width direction of the ventral side exterior sheet, a rear end connected to an inner surface of a central part in the width direction of the dorsal side exterior sheet, and covering from the dorsal side to the ventral side through a crotch part.

## Citation List

## Patent Literature

Patent Literature 1: Japanese Unexamined Patent Publication No. 2008-178682

## Summary of Invention

## Technical Problem

When the underwear-type absorbent article as described above is worn, the pull-up operation of the tubular waistline part (belt) is performed. Here, even when the belt is pulled up, a pulling force is less likely to be transmitted and the belt is less likely to be pulled up in a region of the absorbent body such as a crotch lower part that does not overlap the belt (lower end than the belt). Since the crotch part is not sufficiently pulled up, there is a possibility that a leak or the like occurs.

An aspect of the present invention has been made in view of the above circumstances, and an object of the present invention is to provide an underwear-type absorbent article capable of appropriately pulling up a crotch part in a pull-up operation at the time of wearing and suppressing occurrence of a leakage or the like.

## Solution to Problem

An underwear-type absorbent article according to an aspect of the present invention includes: an absorbent body including a liquid-absorbent absorbent core; a belt part including a ventral waistline part located on one end side in a longitudinal direction of the absorbent body and a dorsal waistline part located on another end side in the longitudinal direction of the absorbent body; and a plurality of crotch elastic members disposed on the absorbent core and extending in the longitudinal direction, in which at least one or more of the crotch elastic members are disposed in each of three regions divided equally in a width direction intersecting the longitudinal direction in the absorbent core.

In the underwear-type absorbent article according to an aspect of the present invention, the plurality of crotch elastic members extending in the longitudinal direction is disposed in the absorbent core. According to such a configuration, in the pull-up operation of pulling up the belt part at the time of wearing, a pull-up force is easily transmitted to a crotch part including the absorbent core via the crotch elastic members. Then, in the underwear-type absorbent article according to an aspect of the present invention, one or more crotch elastic members are disposed in each of the three regions (that is, a central region and left and right end regions) divided equally in the width direction in the absorbent core, and the crotch elastic members are disposed in a wide region in the width direction, so that the crotch part including the absorbent core can be pulled up in a plane as a whole in the pull-up operation. As described above, according to the underwear-type absorbent article according to one aspect of the present invention, the crotch part can be pulled up as a whole in the pull-up operation at the time of wearing, and the occurrence of leakage and the like can be effectively suppressed. Such an effect is more remarkably exhibited, for example, with respect to a sleeping wearer such as a newborn infant. That is, for a sleeping wearer such as a newborn infant, it is difficult to pull up the crotch part to a desired position by the pull-up operation, but since the crotch elastic members are disposed in each of three regions divided equally in the width direction in the absorbent core, the pull-up performance in the pull-up operation is improved, and even for a sleeping wearer such as a newborn infant, the crotch part can be pulled up as a whole, and the occurrence of leakage and the like can be effectively suppressed.

The plurality of crotch elastic members may be disposed so as to straddle a center line that is a bifold line crossing the longitudinal direction in the absorbent core and becomes a crotch lower end part when worn. As described above, since the crotch elastic members are disposed so as to straddle the center line serving as the lower end part of the crotch, the lower end part of the crotch can be pulled up via the crotch elastic members in the pull-up operation, and the crotch part can be more effectively pulled up.

The plurality of crotch elastic members may be disposed to be biased toward a side of the ventral waistline part or a side of the dorsal waistline part with respect to the center line. Regarding the absorbent core, one of the side of ventral waistline part and the side of the dorsal waistline part may be difficult to pull up. In this regard, the crotch elastic members are disposed to be biased toward a side that is difficult to pull up among the side of the ventral waistline part and the side of the dorsal waistline part, so that the side that is difficult to pull up can be pulled up intensively through the crotch elastic members. Then, since the crotch elastic members are disposed to be biased toward the side of the ventral waistline part, fitting of the absorbent core near the crotch part can be improved. Furthermore, since the crotch elastic members are disposed to be biased toward the dorsal waistline part, the ease of pulling up the back body in the lying posture can be improved.

The plurality of crotch elastic members may be disposed to be biased toward a side where a contraction force is relatively low among the ventral waistline part and the dorsal waistline part. Of the ventral waistline part and the dorsal waistline part, the side having a relatively low contraction force is difficult to pull up in conjunction with the pull-up operation at the time of wearing. In this respect, for example, by disposing the crotch elastic members while being biased toward the side where the contraction force is low, the interlocking property with the pull-up operation can be intensively increased on the side where the contraction force is low, and the crotch elastic members can be effectively pulled up in the pull-up operation.

The plurality of crotch elastic members may be disposed so as not to overlap any of the ventral waistline part and the dorsal waistline part, or disposed so as to overlap one of the ventral waistline part and the dorsal waistline part and not to overlap the other. In a case where the crotch elastic members extend to a region overlapping the belt part, there is a possibility that a force is applied in a pulling down direction to the belt part via the crotch elastic members after wearing. In this regard, since the crotch elastic members are disposed so as not to overlap at least both the ventral waistline part and the dorsal waistline part constituting the belt part, even if a force in the pulling down direction is applied to the crotch elastic members, the force in the pulling down direction is suppressed from being strongly transmitted to the belt part, so that the belt part can be effectively suppressed from being pulled down.

A central part of the absorbent core in the longitudinal direction is formed in a constricted shape recessed inward in the width direction, and in a state where the absorbent body is unfolded, an effective length of the crotch elastic members in the longitudinal direction may be shorter than a length of a part formed in the constricted shape in the longitudinal direction. As described above, by making the effective length of the crotch elastic members shorter than the constricted shape of the central part in the longitudinal direction, the vicinity of the constricted shape of the central part in the longitudinal direction can be easily contracted to effectively raise the crotch part, and it is possible to appropriately suppress the crotch elastic members from reaching both end parts in the longitudinal direction and lowering the belt part.

At a center line that is a bifold line crossing the longitudinal direction in the absorbent core and becomes a crotch lower end part when worn, a distance from the crotch elastic member at an outermost end in the width direction included in the plurality of crotch elastic members to an end part in the width direction of the absorbent core may be shorter than a separation distance between two of the crotch elastic members adjacent to each other included in the plurality of crotch elastic members. According to such a configuration, since the crotch elastic member is present at a position close to the end part in the width direction of the absorbent core, the crotch part including the absorbent core can be pulled up in a plane as a whole in the pull-up operation.

The absorbent body may include a leg circumference elastic member stretchable in the longitudinal direction at both end parts of the absorbent body in the width direction, and
the crotch elastic members may have a higher elastic modulus than the leg circumference elastic member. By using such a crotch elastic member, a contraction force of the crotch part increases, and the crotch part can be effectively pulled up.

A pull-up design indicating a pull-up point at the time of wearing may be formed in at least one of a region where the absorbent body and the ventral waistline part overlap each other and a region where the absorbent body and the dorsal waistline part overlap each other. According to such a configuration, the pull-up design is formed in the region where the waistline part and the absorbent body overlap each other, that is, in the region where a pulling up force is easily transmitted to the absorbent body (absorbent core) in the operation of pulling up the belt part, so that a user can be urged to appropriately pull up the crotch part including the absorbent core.

The pull-up design may be formed on a line in the longitudinal direction of a maximum width part in the width direction of the absorbent core. According to such a configuration, in the pull-up operation, the pull-up design is formed in the region where the crotch part including the absorbent core can be pulled up in a plane as a whole, so that the user can be urged to appropriately pull up the crotch part including the absorbent core.

The pull-up design may be formed at an end part of the absorbent core in the longitudinal direction. According to such a configuration, since the pull-up design is formed in the region where the absorbent core itself can be directly pulled up, the user can be urged to appropriately pull up the crotch part including the absorbent core.

The pull-up design may be continuously formed along the longitudinal direction from a region where the absorbent body and the ventral waistline part overlap to a region where the absorbent body and the dorsal waistline part overlap. According to such a configuration, since the pull-up design is continuously formed including the crotch part, the user can easily image the correct pull-up direction.

A plurality of the pull-up designs may be formed apart from each other in the width direction. According to such a configuration, since the pull-up design can be formed at gripping positions of both hands in the actual pull-up operation, it is possible to prompt the user to perform an appropriate pull-up operation.

A pair of the pull-up designs may be disposed on outer sides of the plurality of crotch elastic members so as to sandwich the plurality of crotch elastic members in the width direction. According to such a configuration, the pull-up designs are formed in the region where the force can be appropriately transmitted to all of the plurality of crotch elastic members, that is, the region where the crotch part including the absorbent core can be pulled up in a plane as a whole, so that the user can be urged to appropriately pull up the crotch part including the absorbent core.

A separation distance of the pair of pull-up designs may be twice or less a separation distance of the crotch elastic member at an outermost end in the width direction included in the plurality of crotch elastic members. According to such a configuration, since the separation distance between the crotch elastic member and the pull-up design can be made close in the width direction, in a case where the pull-up operation is performed according to the pull-up designs, the pull-up force can be appropriately transmitted to the crotch elastic members, and the crotch part can be effectively pulled up via the crotch elastic members.

A plurality of the crotch elastic members is disposed in each of two regions divided equally in the width direction in the absorbent core. As described above, since the plurality of crotch elastic members are disposed in each of two regions equally divided in the width direction, a sufficient pulling force in each region can be secured, and the crotch part including the absorbent core can be pulled up in a plane as a whole.

An underwear-type absorbent article according to an aspect of the present invention includes: an absorbent body including a liquid-absorbent absorbent core; a belt part including a ventral waistline part located on one end side in a longitudinal direction of the absorbent body and a dorsal waistline part located on another end side in the longitudinal direction of the absorbent body; and a plurality of crotch elastic members disposed on the absorbent core and stretchable in the longitudinal direction, in which the underwear-type absorbent article has a first size, a dimension of the absorbent body when stretched is a same as that of another underwear-type absorbent article of a second size, and a dimension of the belt part when stretched is a same as that of the other underwear-type absorbent article, and at least one of a number of the crotch elastic members, a contraction force, and a disposition area is larger than those of the other underwear-type absorbent article. In the underwear-type absorbent article according to an aspect of the present invention, the dimension of the absorbent body at the time of stretching and the dimension of the belt part at the time of stretching are the same as those of the other underwear-type absorbent article of the second size, and at least one of the number of the crotch elastic members, the contraction force, and the disposition area is larger than those of the other underwear-type absorbent article. According to such a configuration, at least one of the number of the crotch elastic members, the contraction force, and the disposition area is increased while using the same model paper dimension as the other underwear-type absorbent article of the second size, whereby the underwear-type absorbent article of the first size smaller than the second size can be provided. As described above, since the size of the underwear-type absorbent article is adjusted by the crotch elastic members, it is possible to provide the underwear-type absorbent article having a shape suitable for a size of the wearer while improving the pull-up property of the crotch part by the crotch elastic members.

**Advantageous Effects of Invention**

According to one aspect of the present invention, it is possible to appropriately pull up the crotch part in the pull-up operation at the time of wearing and to suppress the occurrence of a leakage or the like.

**Brief Description of Drawings**

FIG. 1 is a perspective view of an underwear-type absorbent article according to an embodiment.
FIG. 2 is a plan view of the underwear-type absorbent article according to the embodiment when unfolded and stretched as viewed from a non-skin surface side.
FIG. 3 is a plan view of the underwear-type absorbent article according to the embodiment when the underwear-type absorbent article is unfolded and stretched as viewed from a skin surface side.
FIG. 4 is a plan view of an underwear-type absorbent article according to a modified example when the underwear-type absorbent article is unfolded and stretched as viewed from the non-skin surface side.
FIG. 5 is a plan view of an underwear-type absorbent article according to a modified example when the underwear-type absorbent article is unfolded and stretched as viewed from the non-skin surface side.

**Description of Embodiments**

Hereinafter, an underwear-type absorbent article according to an embodiment of the present invention will be described in detail with reference to the drawings. In the drawings, the same or corresponding parts are denoted by the same reference signs, and redundant description will be omitted. Note that the drawings are schematic, and ratios of dimensions and the like may be different from actual ones.

[Overall Schematic Configuration of Underwear-type Absorbent Article]

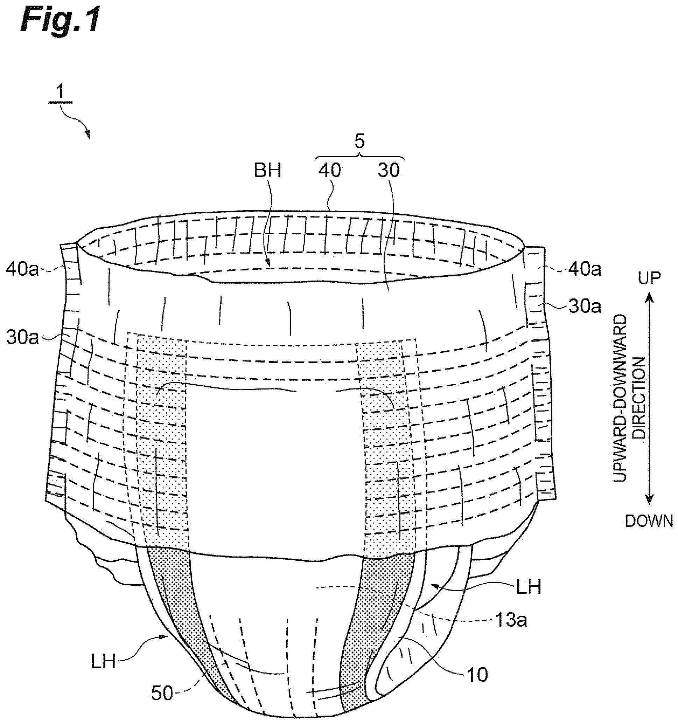

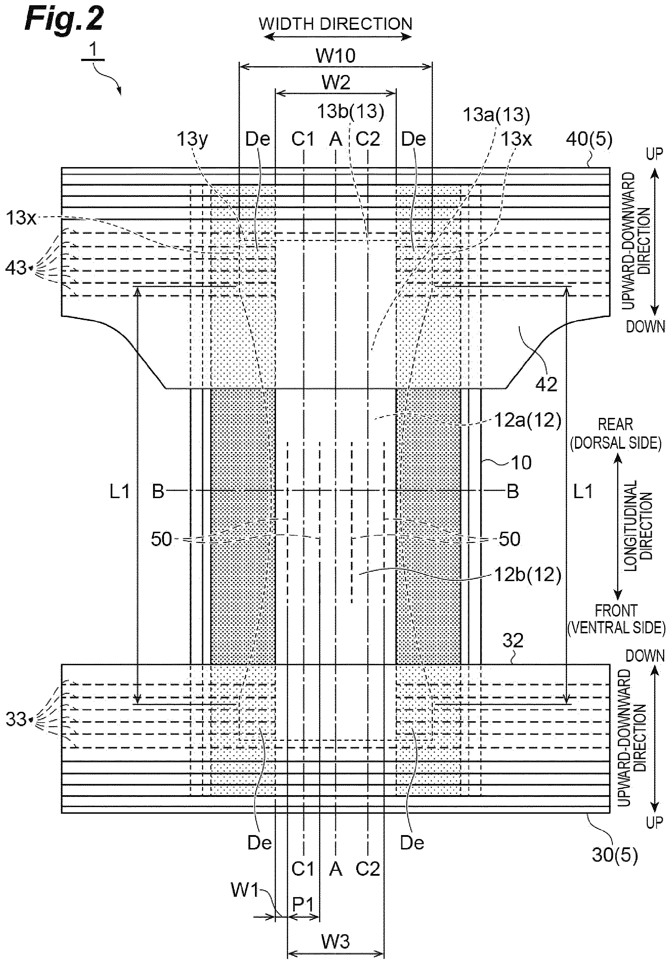

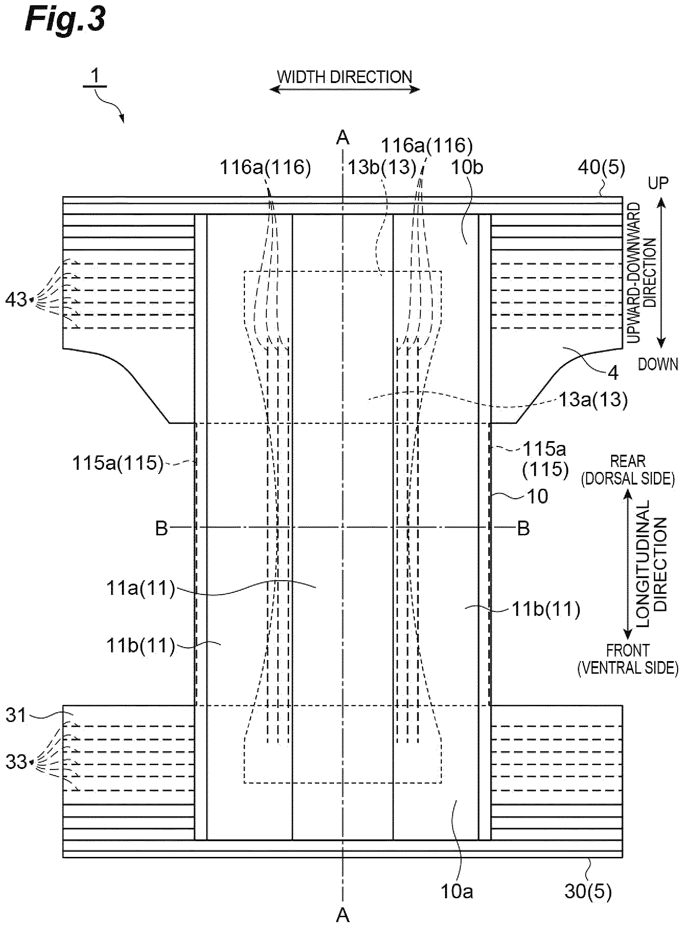

FIG. 1 is a perspective view of an underwear-type absorbent article 1 according to the present embodiment. FIG. 2 is a plan view of the underwear-type absorbent article 1 according to the present embodiment when unfolded and stretched as viewed from the non-skin surface side. FIG. 3 is a plan view of the underwear-type absorbent article 1 according to the present embodiment when the underwear-type absorbent article 1 is unfolded and stretched as viewed from the skin surface side. The "unfolded" state (unfolded state) is a state in which the joining of a side part 30a (see FIG. 1) of a ventral waistline part 30 and a side part 40a (see FIG. 1) of a dorsal waistline part 40 is separated, and the entire underwear-type absorbent article 1 is planarly unfolded. The "stretched" state (stretched state) is a state in which the underwear-type absorbent article 1 is stretched to a state in which no wrinkle is formed. More specifically, the stretched state is a state in which each member (for example, the ventral waistline part 30 and the like) constituting the underwear-type absorbent article 1 is stretched until the dimension thereof matches or approximates the dimension of the single member. In the following description, the "skin surface side" is a side facing the skin of the wearer in the usage state (wearing state) of the underwear-type absorbent article 1, and the "non-skin surface side" is a side facing the side opposite to the skin of the wearer in the usage state (wearing state) of the underwear-type absorbent article 1. Furthermore, the "wearer" is a person wearing the underwear-type absorbent article 1, and the "wearer" is a person wearing the underwear-type absorbent article 1.

Furthermore, in the following description, an upward-downward direction in the worn state of the underwear-type absorbent article 1 (see FIG. 1) may be referred to as "upward-downward direction", the longitudinal direction in the stretched state of the underwear-type absorbent article 1 (see FIGS. 2 and 3) may be referred to as "longitudinal direction", and the width direction in the stretched state of the underwear-type absorbent article 1 (see FIGS. 2 and 3), which is a direction orthogonal to the longitudinal direction, may be referred to as "width direction". The "longitudinal direction" is a direction extending from the front side to the rear side of the body of the wearer in the wearing state of the underwear-type absorbent article 1. One side in the longitudinal direction may be referred to as a "ventral side", and the other side may be referred to as a "dorsal side". The "width direction" is a direction that is the left-right direction of the wearer in the wearing state of the underwear-type absorbent article 1.

The underwear-type absorbent article 1 is, for example, an underwear-type disposable diaper. The underwear-type absorbent article 1 may be an underwear-type disposable diaper for children or an underwear-type disposable diaper for adults. In the present embodiment, an example in which the underwear-type absorbent article 1 is an underwear-type disposable diaper for children, particularly for newborns will be described.

The underwear-type absorbent article 1 has a so-called three-piece configuration, and includes an absorbent body 10, a ventral waistline part 30, and a dorsal waistline part 40. Furthermore, the underwear-type absorbent article 1 includes a plurality of crotch elastic members 50 disposed on an absorbent core 13a of the absorbent body 10 and extending in the longitudinal direction. Details of the crotch elastic member 50 will be described later. The ventral waistline part 30 and the dorsal waistline part 40 have a substantially rectangular shape in plan view, and the extending direction thereof is along the width direction. The ventral waistline part 30 covers the ventral side of the wearer, and the dorsal waistline part 40 covers the dorsal side of the wearer. The absorbent body 10 has a substantially rectangular shape in plan view, and its extending direction is along the longitudinal direction of the underwear-type absorbent article 1.

In the underwear-type absorbent article 1, the ventral waistline part 30 is positioned at a ventral end part 10a (see FIG. 3) that is one end side in the longitudinal direction of the absorbent body 10, and the dorsal waistline part 40 is positioned at a dorsal end part 10b (see FIG. 3) that is the other end side in the longitudinal direction of the absorbent body 10. Then, the side surface on the non-skin surface side of the ventral end part 10a and the ventral waistline part 30 are joined by an adhesive or the like (not illustrated), and the side surface on the non-skin surface side of the dorsal end part 10b and the dorsal waistline part 40 are joined by an adhesive or the like (not illustrated). In this state, as illustrated in FIG. 1, the absorbent body 10 is folded in two such that the ventral waistline part 30 and the dorsal waistline part 40 face each other, and the side part 30a of the ventral waistline part 30 in the width direction and the side part 40a of the dorsal waistline part 40 in the width direction are welded and joined to each other, whereby the underwear-type absorbent article 1 is formed in a pants shape. The ventral waistline part 30 and the dorsal waistline part 40 constitute a belt part 5. As illustrated in FIG. 1, a waistline opening BH and a pair of leg openings LH are formed in the underwear-type absorbent article 1.

As illustrated in FIGS. 2 and 3, the ventral waistline part 30 includes a skin surface sheet 31, a non-skin surface sheet 32, and a plurality of thread-like elastic members 33. The non-skin surface sheet 32 is disposed on the non-skin surface side of the skin surface sheet 31. The skin surface sheet 31 and the non-skin surface sheet 32 are disposed on the non-skin surface side of the absorbent body 10. As the skin surface sheet 31 and the non-skin surface sheet 32, for example, a non-stretchable sheet such as an SMS nonwoven fabric (spunbond meltblown spunbond nonwoven fabric), a spunbond nonwoven fabric, an air-through nonwoven fabric, a plastic sheet, an open hole plastic sheet, or a laminate sheet thereof is used. The skin surface sheet 31 and the non-skin surface sheet 32 are bonded to each other by an adhesive such as hot melt. The plurality of thread-like elastic members 33 are disposed side by side in the upward-downward direction between the skin surface sheet 31 and the non-skin surface sheet 32, and are fixed in a stretched state in the width direction. As a result, the ventral waistline part 30 expands and contracts in the width direction and fits the waistline of the wearer. As the thread-like elastic member 33, for example, an elastic member such as rubber or spandex is used. Note that, in the ventral waistline part 30, as illustrated in FIG. 2, the thread-like elastic member 33 may be disposed such that the thread-like elastic member 33 is not provided in a part of a region overlapping the absorbent core 13a (details will be described later) of the absorbent body 10. That is, the thread-like elastic member 33 may be cut halfway in the width direction. As a result, it is possible to suppress the influence of expansion and contraction in the width direction of the thread-like elastic member 33 for fitting the ventral waistline part 30 to the waistline from being exerted on the disposition or the like of the absorbent core 13a.

As illustrated in FIGS. 2 and 3, the dorsal waistline part 40 includes a skin surface sheet 41, a non-skin surface sheet 42, and a plurality of thread-like elastic members 43. The non-skin surface sheet 42 is disposed on the non-skin surface side of the skin surface sheet 41. The skin surface sheet 41 and the non-skin surface sheet 42 are disposed on the non-skin surface side of the absorbent body 10. As the skin surface sheet 41 and the non-skin surface sheet 42, for example, a non-stretchable sheet such as an SMS nonwoven fabric (spunbond meltblown spunbond nonwoven fabric), a spunbond nonwoven fabric, an air-through nonwoven fabric, a plastic sheet, an open hole plastic sheet, or a laminate sheet thereof is used. The skin surface sheet 41 and the non-skin surface sheet 42 are bonded to each other by an adhesive such as hot melt. The plurality of thread-like elastic members 43 are disposed side by side in the upward-downward direction between the skin surface sheet 41 and the non-skin surface sheet 42, and are fixed in a stretched state in the width direction. As a result, the dorsal waistline part 40 expands and contracts in the width direction and fits the waistline of the wearer. As the thread-like elastic member 43, for example, an elastic member such as rubber or spandex is used. Note that, in the dorsal waistline part 40, as illustrated in FIG. 2, the thread-like elastic member 43 may be disposed such that the thread-like elastic member 43 is not provided in a part of a region overlapping the absorbent core 13a (details will be described later) of the absorbent body 10. That is, the thread-like elastic member 43 may be cut halfway in the width direction. As a result, it is possible to suppress the influence of expansion and contraction in the width direction of the thread-like elastic member 43 for fitting the dorsal waistline part 40 to the waistline from being exerted on the disposition or the like of the absorbent core 13a.

As illustrated in FIGS. 2 and 3, the absorbent body 10 includes a skin surface sheet 11, a non-skin surface sheet 12, and an absorber 13.

The absorber 13 includes the liquid-absorbent absorbent core 13a and a core wrap 13b. The absorbent core 13a is configured to take in excrement, and is, for example, a laminate of an absorbent material containing ground pulp, a super absorbent polymer (SAP), or a mixture thereof. The absorbent core 13a has a maximum length in the width direction at both end parts in the longitudinal direction, and is formed in a constricted shape recessed inward in the width direction toward the central part in the longitudinal direction. The core wrap 13b is disposed so as to cover the skin surface side and the non-skin surface side of the absorbent core 13a, and is, for example, a tissue or a nonwoven fabric sheet. The absorber 13 is provided in substantially the entire region in the longitudinal direction except for both end parts in the longitudinal direction at the central part in the width direction of the absorbent body 10.

As illustrated in FIG. 3, the skin surface sheet 11 includes a surface sheet 11a and a side sheet 11b. The surface sheet 11a is a liquid-permeable sheet disposed on the skin surface side so as to cover the absorber 13, and allows excreta to pass in the direction of the absorber 13. The surface sheet 11a is made of, for example, a liquid-permeable nonwoven fabric. The side sheet 11b is a liquid impervious sheet disposed in the entire region in the longitudinal direction so as to cover both side parts of the surface sheet 11a in the width direction outside the central part in the width direction of the absorber 13. Both end parts of the side sheet 11b in the longitudinal direction are fixed to the surface sheet 11a by bonding using an adhesive or the like. Note that the method of fixing the side sheet 11b to the surface sheet 11a is not limited to bonding, and may be ultrasonic bonding, thermal fusion by embossing rolls, or the like. The side sheet 11b is made of, for example, a hydrophobic nonwoven fabric.

As illustrated in FIG. 3, a plurality of (for example, three) thread-like elastic members 116a are provided along the longitudinal direction at the inner end part in the width direction of the side sheet 11b. As the thread-like elastic member 116a, for example, an elastic member such as rubber or spandex is used. The front end of the thread-like elastic member 116a reaches the region of the ventral waistline part 30, and the rear end thereof reaches the region of the dorsal waistline part 40. Furthermore, one or a plurality of thread-like elastic members 115a are provided along the longitudinal direction at an outer end part in the width direction of the side sheet 11b. The thread-like elastic member 115a is a leg circumference elastic member that is stretchable in the longitudinal direction at both end parts thereof in the width direction. As the thread-like elastic member 115a, for example, an elastic member such as rubber or spandex is used. The front end of the thread-like elastic member 115a reaches the vicinity of the ventral waistline part 30, and the rear end thereof reaches the vicinity of the dorsal waistline part 40.

As illustrated in FIG. 2, the non-skin surface sheet 12 includes a back surface sheet 12a and an outer nonwoven fabric 12b. The back surface sheet 12a is a liquid impervious sheet disposed on the non-skin surface side so as to cover the absorber 13, and is configured to prevent excreta from leaking to the outside. The back surface sheet 12a is made of, for example, a film. The outer nonwoven fabric 12b is a liquid impervious sheet disposed on the non-skin surface side of the back surface sheet 12a. The outer nonwoven fabric 12b is made of, for example, a hydrophobic nonwoven fabric.

As illustrated in FIG. 3, the absorbent body 10 includes at least a leg stretchable part 115 and a leg side gather 116 as a functional configuration thereof.

The leg stretchable part 115 is configured to include a thread-like elastic member 115a which is a stretchable part stretchable in the longitudinal direction on both side parts (both end parts in the width direction) of the absorbent body 10. In the leg stretchable part 115, the thread-like elastic members 115a contract to form leg gathers that improve fitting around the wearer's legs in the crotch region in the used state.

The leg side gather 116 is provided inside the leg stretchable part 115 in the width direction, has both end parts in the longitudinal direction fixed, and is configured to be able to stand by contracting in the longitudinal direction. The leg side gather 116 includes the side sheets 11b described above. In the leg side gather 116, the thread-like elastic member 116a contracts in a state where both end parts of the side sheet 11b are fixed to the surface sheet 11a, so that a pair of leakage-preventing cuffs standing up toward the skin of the wearer and preventing leakage of excreta along the surface of the surface sheet 11a is formed in the crotch region in the used state.

[Details of Crotch Elastic Member]

As illustrated in FIG. 2, the underwear-type absorbent article 1 includes a plurality of (for example, four) crotch elastic members 50. The plurality of crotch elastic members 50 is disposed on the absorbent core 13a and extends in the longitudinal direction. Here, "disposed on the absorbent core 13a" means disposed so as to overlap the absorbent core 13a at least in the thickness direction of the absorbent body 10. In the present embodiment, each crotch elastic member 50 is disposed on the non-skin surface side of the back surface sheet 12a of the non-skin surface sheet 12 and on the skin surface side of the outer nonwoven fabric 12b and in a region overlapping the absorbent core 13a in the thickness direction. As the crotch elastic member 50, for example, an elastic member such as rubber or spandex is used. Furthermore, as the crotch elastic member 50, a stretchable film or the like may be used. The crotch elastic member 50 has a higher elastic modulus than the above-described thread-like elastic member 115a that functions as a leg circumference elastic member. Furthermore, the crotch elastic member 50 may have a higher elastic modulus than the thread-like elastic member 116a. The elastic modulus is determined by, for example, the thickness × magnification of the elastic member.

Note that the "elastic modulus" mentioned here is an elastic modulus or an elastic constant mainly focusing on expansion and contraction of the underwear-type absorbent article 1 in the longitudinal direction, and corresponds to the following Young's modulus E.

$$E = \sigma/\varepsilon$$

$\sigma$: Stress due to elongation of elastic member
$\varepsilon$: Distortion due to elongation of elastic member

As illustrated in FIG. 2, in a state where the absorbent body 10 is unfolded, an effective length in the longitudinal direction of the crotch elastic member 50 is shorter than a length L1 in the longitudinal direction of a part formed in a constricted shape of the absorbent core 13a.

The plurality of crotch elastic members 50 extend in the longitudinal direction so as to straddle a center line B-B which is a bifold line crossing the longitudinal direction of the absorbent core 13a and becomes a crotch lower end part at the time of wearing. As illustrated in FIG. 2, the plurality of crotch elastic members 50 is disposed to be biased toward a side of the ventral waistline part 30 side with reference to the center line B-B. That is, in the plurality of crotch elastic members 50, an effective length of a part extending in the ventral waistline part 30 direction is longer than an effective length of a part extending in the dorsal waistline part 40 direction with reference to the center line B-B. Note that the plurality of crotch elastic members 50 may be disposed to be biased to a side where the contraction force is relatively low among the ventral waistline part 30 and the dorsal waistline part 40.

The plurality of crotch elastic members 50 are disposed so as not to overlap with any of the ventral waistline part 30 and the dorsal waistline part 40. That is, as illustrated in FIG. 2, each crotch elastic member 50 does not reach a region where the front end part (ventral side end part) in the longitudinal direction overlaps the ventral waistline part 30, and does not reach a region where the rear end part (dorsal side end part) in the longitudinal direction overlaps the dorsal waistline part 40. Note that, in the present embodiment, it is described that each crotch elastic member 50 is disposed so as not to overlap with either the ventral waistline part 30 or the dorsal waistline part 40, but each crotch elastic member 50 may be disposed so as to overlap one of the ventral waistline part 30 and the dorsal waistline part 40 and not to overlap the other.

At least one or more crotch elastic members 50 are disposed in each of three regions divided equally in the width direction in the absorbent core 13a. As illustrated in FIG. 2, each of three regions divided equally in the width direction in the absorbent core 13a is, for example, a region defined by trisecting lines C1 to C1 and C2 to C2 that divide the maximum length W10, which is the length between both end parts 13x and 13x in the maximum width part of the absorbent core 13a in the width direction, into three equal parts. In the example illustrated in FIG. 2, one crotch elastic member 50 is disposed in a region on the left side of the trisection line C1 to C1, two crotch elastic members 50 are disposed in a region between the trisection line C1 to C1 and the trisection line C2 to C2, and one crotch elastic member 50 is disposed in a region on the right side of the trisection line C2 to C2. Note that, in the present embodiment, each of three regions divided equally in the width direction is described as a region obtained by dividing the maximum length W10 into three equal parts as described above, but the region divided into three equal parts in the width direction may be a region obtained by dividing a region other than the maximum width part of the absorbent core 13a into three equal parts in the width direction.

Furthermore, a plurality of crotch elastic members 50 may be disposed in each of two regions divided equally in the width direction in the absorbent core 13a. As illustrated in FIG. 2, each of two regions divided equally in the width direction is, for example, a region defined by a bisector line A-A that bisects a maximum length W10 which is a length between both end parts 13x and 13x in the maximum width part of the absorbent core 13a in the width direction. In the example illustrated in FIG. 2, two crotch elastic members 50 are disposed in a region on the left side of the bisector A-A, and two crotch elastic members 50 are disposed in a region on the right side of the bisector A-A. Note that the plurality of crotch elastic members 50 may be evenly (at equal intervals) disposed in the width direction of the absorbent core 13a. By arranging the plurality of crotch elastic members 50 at equal intervals in this manner, the absorbent core 13a can be pulled up in a plane.

Since the plurality of crotch elastic members 50 is disposed in each of three regions divided equally in the width direction as described above, the plurality of crotch elastic members are also disposed at both end parts in the width direction. Specifically, in the center line B-B serving as the lower end part of the crotch, the crotch elastic member 50 at the outermost end in the width direction is disposed on the end side in the width direction to such an extent that a distance W1 from the crotch elastic member 50 at the outermost end in the width direction to the end in the width direction of the absorbent core 13a is shorter than a crotch elastic member pitch P1 which is a separation distance between two adjacent crotch elastic members 50 and 50 included in the plurality of crotch elastic members. Note that, as the distance W1, there are a distance from the crotch elastic member 50 at the left end illustrated in FIG. 2 to the left end of the absorbent core 13a and a distance from the crotch elastic member 50 at the right end illustrated in FIG. 2 to the right end of the absorbent core 13a. In a case where these are values different from each other, the distance W1 may be any one of any values, may be a value with a shorter distance, may be a value with a longer distance, or may be an average value of the respective values. Furthermore, three patterns of the crotch elastic member pitch P1 are conceivable in the example illustrated in FIG. 2, but in a case where these values are different from each other, any one value may be used, a value of a part having the shortest pitch may be used, a value of a part having the longest distance may be used, or an average value of each pitch may be used.

[Details of Pull-up Design]

As illustrated in FIG. 2, a pull-up design De indicating a pull-up point of the wearer at the time of wearing is formed in a region where the absorbent body 10 and the ventral waistline part 30 overlap and a region where the absorbent body 10 and the dorsal waistline part 40 overlap. The pull-up design De is, for example, drawn on the back surface sheet 12a of the absorbent body 10, and is formed so as to be visible to the wearer at the time of wearing. The pull-up design De may be any design as long as it can indicate the pull-up point to the wearer (indicate the pull-up point separately from other regions), and may be indicated in a color different from that of the other regions, may be indicated by a figure such as a square, or may be indicated by a simple linear pattern. Furthermore, in a case where the pull-up design De includes a linear pattern, the line may be a horizontal line, a vertical line, or a diagonal line.

Two (a pair of) pull-up designs De are formed apart from each other in the width direction, one pull-up design De is formed on one end side (left end side) in the width direction of the back surface sheet 12a, and the other pull-up design De is formed on the other end side (right end side) in the width direction of the back surface sheet 12a. Each pull-up design De is formed continuously (on a straight line) along the longitudinal direction from a region where the absorbent body 10 and the ventral waistline part 30 overlap to a region where the absorbent body 10 and the dorsal waistline part 40 overlap. That is, the pull-up design De is formed not only in the region where the absorbent body 10 and the ventral waistline part 30 (and the dorsal waistline part 40) overlap each other but also in substantially the entire region in the longitudinal direction of the absorbent body 10 including the crotch lower part and the like.

Note that, in FIG. 2, in the pull-up design De, the hatching in the region where the absorbent body 10 and the ventral waistline part 30 (and the dorsal waistline part 40) overlap is relatively thin, and the hatching in the region such as the crotch lower part is relatively dark.

This expresses that the pull-up design De is visually recognized through the ventral waistline part 30 (or the dorsal waistline part 40) in the region where the absorbent body 10 and the ventral waistline part 30 (and the dorsal waistline part 40) overlap, and thus the wearer visually recognizes that the pull-up design De is relatively thin.

The pair of pull-up designs De and De formed to be separated from each other in the width direction are disposed outside the plurality of crotch elastic members 50 so as to sandwich the plurality of crotch elastic members 50 in the width direction. The separation distance W2 of the pair of pull-up designs De and De is set to be equal to or less than twice the separation distance W3 of the outermost crotch elastic members 50 and 50 in the width direction included in the plurality of crotch elastic members 50. In this way, by reducing the difference between the separation distance W2 and the separation distance W3 to about two times or less, the separation distance between the crotch elastic member 50 and the pull-up design De can be brought close.

Furthermore, the pull-up designs De and De are formed on a line in the longitudinal direction of the maximum width part (part indicated by the maximum width length W10) in the width direction of the absorbent core 13a. In such a configuration, the pull-up designs De and De are formed in a region where the absorbent core 13a can be pulled up in a plane as a whole.

Furthermore, the pull-up designs De and De are formed at an end part 13y in the longitudinal direction of the absorbent core 13a. In such a configuration, the pull-up designs De and De are formed in a region where the absorbent core 13a itself can be directly pulled up.

[Dimensions of Model Paper of Underwear-type Absorbent Article]

The underwear-type absorbent article 1 according to the present embodiment is, for example, an underwear-type disposable diaper for a baby. The underwear-type absorbent article 1 has a size for a neonate (first size). For example, the other underwear-type absorbent article having a so-called S size (second size) and the absorbent body 10 have the same dimensions when stretched, and the ventral waistline part 30 and the dorsal waistline part 40 constituting the belt part 5 have the same dimensions when stretched. Here, the "other underwear-type absorbent article" is, for example, an underwear-type absorbent article having the same brand name and sub-brand name as the underwear-type absorbent article 1 and having another size (for example, S size (second size)) used for infants larger than newborns. The "other underwear-type absorbent article" here has the same basic configuration as the underwear-type absorbent article 1, and may also include an elastic member corresponding to the crotch elastic member 50. Note that the "other underwear-type absorbent article" may not include the elastic member corresponding to the crotch elastic member 50. In the underwear-type absorbent article 1, for example, at least one of the number of the crotch elastic members 50, the contraction force, and the disposition area is increased as compared with the "other underwear-type absorbent article" of S size. As described above, in the underwear-type absorbent article 1, at least one of the number of the crotch elastic members 50, the contraction force, and the disposition area is increased, so that it can be used as an underwear-type disposable diaper having a size for newborns smaller than S size by size adjustment using the crotch elastic members 50 while using the same model paper dimension as the "other underwear-type absorbent articles". Note that the "other underwear-type absorbent article" may be different in brand name (or sub-brand name) from the underwear-type absorbent article 1.

[Operation and Effect]

Next, functions and effects of the underwear-type absorbent article 1 according to the present embodiment will be described.

An underwear-type absorbent article 1 according to the present embodiment includes: an absorbent body 10 including a liquid-absorbent absorbent core 13a; a belt part 5 including a ventral waistline part 30 located on one end side in a longitudinal direction of the absorbent body 10 and a dorsal waistline part 40 located on the other end side in the longitudinal direction of the absorbent body 10; and a plurality of crotch elastic members 50 disposed on the absorbent core 13a and extending in the longitudinal direction, in which at least one or more of the crotch elastic members 50 are disposed in each of three regions equally divided in a width direction intersecting the longitudinal direction in the absorbent core 13a.

In the underwear-type absorbent article 1 according to the present embodiment, a plurality of crotch elastic members 50 extending in the longitudinal direction is disposed on the absorbent core 13a. According to such a configuration, in the pull-up operation of pulling up the belt part 5 at the time of wearing, the pull-up force is easily transmitted to the crotch part including the absorbent core 13a via the crotch elastic member 50. In the underwear-type absorbent article 1 according to the present embodiment, one or more crotch elastic members 50 are disposed in each of three regions (that is, the central region and the left and right end regions) divided equally in the width direction in the absorbent core 13a, and the crotch elastic members 50 are disposed in a wide area in the width direction, so that the crotch part including the absorbent core 13a can be pulled up in a plane as a whole in the pull-up operation. As described above, according to the underwear-type absorbent article 1 of the present embodiment, the crotch part can be pulled up as a whole in the pull-up operation at the time of wearing, and the occurrence of leakage and the like can be effectively suppressed.

The plurality of crotch elastic members 50 may be disposed so as to straddle a center line B-B which is a bifold line crossing the longitudinal direction of the absorbent core 13a and becomes a crotch lower end part at the time of wearing. As described above, since the crotch elastic member 50 is disposed so as to straddle the center line B-B serving as the lower end part of the crotch, the lower end part of the crotch can be pulled up via the crotch elastic member 50 in the pull-up operation, and the crotch can be more effectively pulled up.

The plurality of crotch elastic members 50 may be disposed to be biased toward the side of the ventral waistline part 30 with reference to the center line B-B. One of the ventral waistline part 30 side and the dorsal waistline part 40 side of the absorbent core 13a may be difficult to pull up. In this regard, the crotch elastic member 50 is disposed so as to be biased to the side that is difficult to pull up among the ventral waistline part 30 side and the dorsal waistline part 40 side, whereby the side that is difficult to pull up can be intensively pulled up via the crotch elastic member 50. Then, since the crotch elastic member 50 is disposed to be biased toward the side of the ventral waistline part 30, fitting of the vicinity of the crotch part of the absorbent core 13a can be improved.

The plurality of crotch elastic members 50 may be disposed to be biased to the side where the contraction force is relatively low in the ventral waistline part 30 and the dorsal waistline part 40. Of the ventral waistline part 30 and the dorsal waistline part 40, the side having a relatively low contraction force is difficult to pull up in conjunction with the pull-up operation at the time of wearing. In this respect, for example, by disposing the crotch elastic member 50 to be biased to the side where the contraction force is low, the interlocking property with the pull-up operation can be intensively increased on the side where the contraction force is low, and the crotch elastic member can be effectively pulled up in the pull-up operation.

The plurality of crotch elastic members 50 may be disposed so as not to overlap either the ventral waistline part 30 or the dorsal waistline part 40, or may be disposed so as to overlap one of the ventral waistline part 30 and the dorsal waistline part 40 and not to overlap the other. In a case where the crotch elastic member 50 extends to a region overlapping the belt part 5, there is a possibility that a force is applied in a pulling direction to the belt part 5 via the crotch elastic member 50 after wearing. In this regard, since the crotch elastic member 50 is disposed so as not to overlap at least both the ventral waistline part 30 and the dorsal waistline part 40 constituting the belt part 5, even if a force in the pulling direction is applied to the crotch elastic member 50, the force in the pulling direction is suppressed from being strongly transmitted to the belt part 5, so that the belt part 5 can be effectively suppressed from being pulled down.

The central part in the longitudinal direction of the absorbent core 13a is formed in a constricted shape recessed inward in the width direction, and in a state where the absorbent body 10 is unfolded, the effective length in the longitudinal direction of the crotch elastic member 50 may be shorter than the length L1 in the longitudinal direction of the part formed in the constricted shape. As described above, by making the effective length of the crotch elastic member shorter than the constricted shape of the central part in the longitudinal direction, the vicinity of the constricted shape of the central part in the longitudinal direction can be easily contracted to effectively raise the crotch part, and it is possible to appropriately suppress the crotch elastic member 50 from reaching both end parts in the longitudinal direction and lowering the belt part 5.

In the center line B-B that is a bifold line intersecting the longitudinal direction of the absorbent core 13a and is the lower end part of the crotch when worn, the distance W1 from the outermost crotch elastic member 50 in the width direction included in the plurality of crotch elastic members 50 to the end part of the absorbent core 13a in the width direction may be shorter than a crotch elastic member pitch P1 that is a separation distance between two adjacent crotch elastic members 50 and 50 included in the plurality of crotch elastic members 50. According to such a configuration, since the crotch elastic member 50 is present at a position close to the end part in the width direction of the absorbent core 13a, the crotch part including the absorbent core 13a can be pulled up in a plane as a whole in the pull-up operation.

The absorbent body 10 may have a thread-like elastic member 115a that functions as a leg circumference elastic member stretchable in the longitudinal direction at both end parts thereof in the width direction, and the crotch elastic member 50 may have a higher elastic modulus than the thread-like elastic member 115a. By using such a crotch elastic member 50, the contraction force of the crotch part increases, and the crotch part can be effectively pulled up.

A pull-up design De indicating a pull-up point at the time of wearing may be formed in at least one of a region where the absorbent body 10 and the ventral waistline part 30 overlap and a region where the absorbent body 10 and the dorsal waistline part 40 overlap. According to such a configuration, the pull-up design is formed in the region where the ventral waistline part 30 and the absorbent body 10 overlap, that is, the region where it is easy to transmit the pulling force to the absorbent body 10 (absorbent core 13a) in the operation of pulling up the belt part 5, and thus, it is possible to encourage the user to appropriately pull up the crotch part including the absorbent core 13a.

The pull-up design De may be formed on a line in the longitudinal direction of the maximum width part (part indicated by the maximum width length W10) in the width direction of the absorbent core 13a. According to such a configuration, in the pull-up operation, the pull-up design De is formed in the region where the crotch part including the absorbent core 13a can be pulled up in a plane as a whole, so that the user can be urged to appropriately pull up the crotch part including the absorbent core 13a.

The pull-up design De may be formed at the end part 13y in the longitudinal direction of the absorbent core 13a. According to such a configuration, since the pull-up design De is formed in the region where the absorbent core 13a itself can be directly pulled up, it is possible to encourage the user to appropriately pull up the crotch part including the absorbent core 13a.

The pull-up design De may be continuously formed along the longitudinal direction from the region where the absorbent body 10 and the ventral waistline part 30 overlap to the region where the absorbent body 10 and the dorsal waistline part 40 overlap. According to such a configuration, since the pull-up design De is continuously formed including the crotch part, the user can easily image the correct pull-up direction.

A plurality (a pair) of the pull-up designs De may be formed apart from each other in the width direction. According to such a configuration, since the pull-up designs De and De can be formed at the holding positions of both hands in the actual pull-up operation, it is possible to prompt the user to perform an appropriate pull-up operation.

The pair of pull-up designs De and De may be disposed outside the plurality of crotch elastic members 50 so as to sandwich the plurality of crotch elastic members 50 in the width direction. According to such a configuration, the pull-up design De is formed in the region where the force can be appropriately transmitted to all of the plurality of crotch elastic members 50, that is, the region where the crotch part including the absorbent core 13a can be pulled up in a plane as a whole. Therefore, it is possible to encourage the user to appropriately pull up the crotch part including the absorbent core 13a.

The separation distance W2 of the pair of pull-up designs De and De may be twice or less the separation distance W3 of the outermost crotch elastic members 50 and 50 in the width direction included in the plurality of crotch elastic members 50. According to such a configuration, since the separation distance between the crotch elastic member 50 and the pull-up design De can be brought close in the width direction, when the pull-up operation is performed according to the pull-up design De, the pull-up force can be appropriately transmitted to the crotch elastic member 50, and the crotch part can be effectively pulled up via the crotch elastic member 50.

A plurality of crotch elastic members 50 may be disposed in each of two regions divided equally in the width direction in the absorbent core 13a. As described above, since the plurality of crotch elastic members 50 is disposed in each of two regions divided equally in the width direction, a sufficient pulling force in each region can be secured, and the crotch part including the absorbent core 13a can be pulled up in a plane as a whole.

The underwear-type absorbent article 1 may have a first size (for example, a size for a baby), have the same dimensions as those of other underwear-type absorbent articles having a second size (for example, S size) when the absorbent body 10 is stretched, and have the same dimensions as those of the ventral waistline part 30 and the dorsal waistline part 40 constituting the belt part 5 when stretched, and may have at least one of the number of the crotch elastic members 50, the contraction force, and the disposition area larger than those of other underwear-type absorbent articles. According to such a configuration, with respect to the absorbent body 10 and the belt part 5, at least one of the number of the crotch elastic members 50, the contraction force, and the disposition area is increased while using the same model paper dimension as the other underwear-type absorbent articles of the second size, whereby the underwear-type absorbent article of the first size smaller than the second size can be provided. As described above, the size of the underwear-type absorbent article is adjusted by the crotch elastic member 50, so that it is possible to provide the underwear-type absorbent article having a shape suitable for the size of the wearer while improving the pull-up property of the crotch part by the crotch elastic member 50. Note that while the underwear-type absorbent article 1 uses the same model paper dimension as the other underwear-type absorbent articles of the second size (for example, S size), for example, when the materials constituting the absorbent body 10 and the belt part 5 are bonded, the materials may be bonded such that the dimension of the finished product (after bonding the materials) at the time of stretching is smaller than the dimension of the finished product of the second size at the time of stretching. That is, in the underwear-type absorbent article 1, the bonding position of the material may be adjusted such that the dimension of the finished product (after bonding of the material) at the time of stretching is smaller than the dimension of the finished product having the second size at the time of stretching.

[Modified Examples]

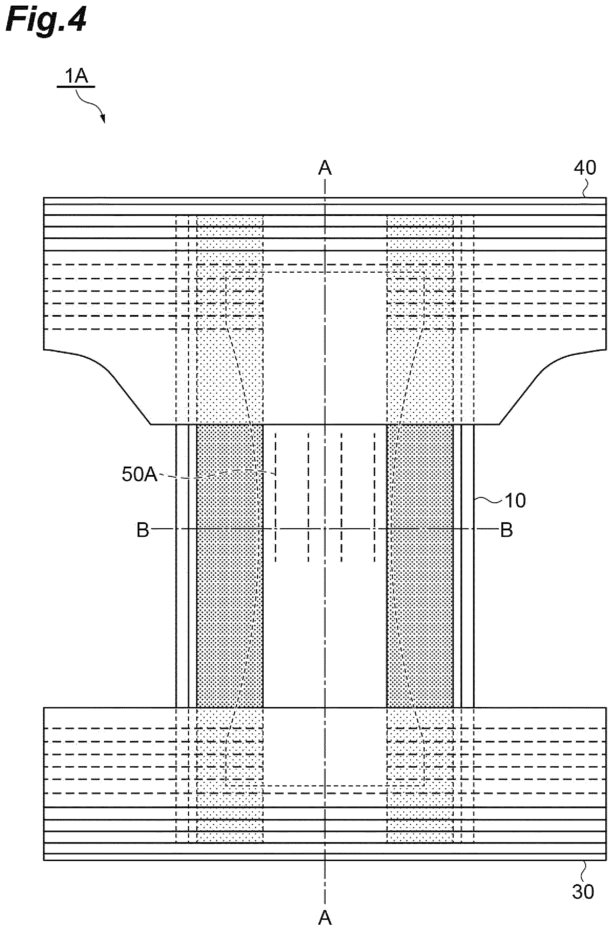

Although the embodiments of the present invention have been described above, the present invention is not limited to the above embodiments. For example, the description has been given assuming that the plurality of crotch elastic members 50 is disposed to be biased to the side of the ventral waistline part 30 with reference to the center line B-B, but the present invention is not limited thereto. For example, in a case where the contraction force of the dorsal waistline part 40 is relatively low, the crotch elastic member 50A may be disposed to be biased to a side of the dorsal waistline part 40 with reference to the center line B-B as in an underwear-type absorbent article 1A illustrated in FIG. 4. Since the crotch elastic member 50A is disposed to be biased toward the side of the dorsal waistline part 40, it is possible to improve the ease of pulling up the back body in the lying posture.

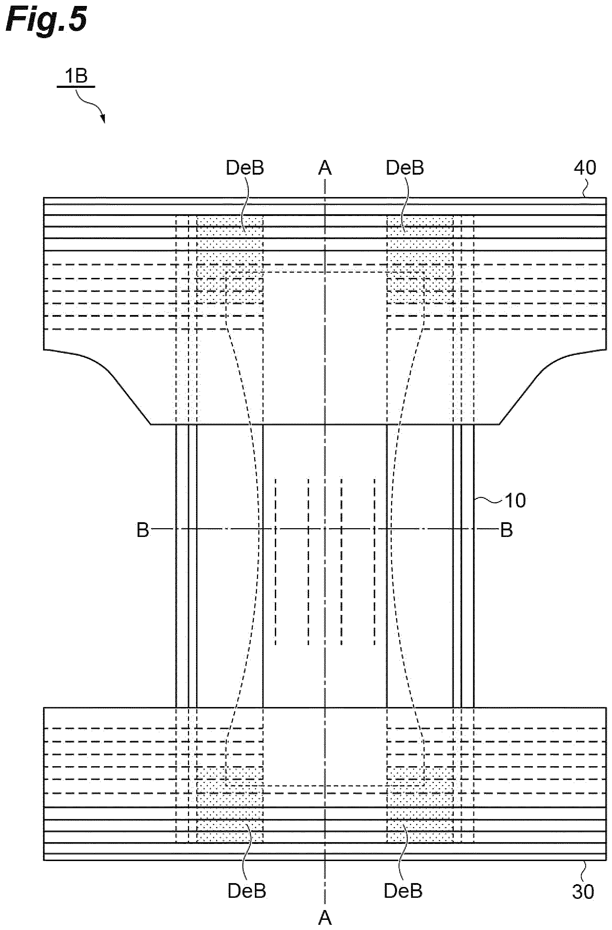

Furthermore, in the above embodiment, the pull-up design De has been described as being continuously formed along the longitudinal direction from the region where the absorbent body 10 and the ventral waistline part 30 overlap to the region where the absorbent body 10 and the dorsal waistline part 40 overlap, but the present invention is not limited thereto. For example, as in an underwear-type absorbent article 1B illustrated in FIG. 5, the pull-up designs DeB, DeB, DeB, and DeB may be formed only at the four corners of the absorbent body 10. Note that even in the configuration in which the pull-up design DeB is formed only at the four corners, the pull-up design DeB is formed on the line in the longitudinal direction of the maximum width part in the width direction of the absorbent core, so that the crotch part including the absorbent core can be pulled up in a plane as a whole in the pull-up operation, and the pull-up design DeB is formed in the region, so that the user can be urged to appropriately pull up the crotch part including the absorbent core. Furthermore, even in the configuration in which the pull-up design DeB is formed only at the four corners, since the pull-up design DeB is formed at the end part of the absorbent core in the longitudinal direction, the pull-up design DeB is formed in a region where the absorbent core itself can be directly pulled up, so that it is possible to encourage the user to appropriately pull up the crotch part including the absorbent core. Furthermore, in the above embodiment, it has been described that the pull-up design De may be formed at the end part 13y in the longitudinal direction of the absorbent core 13a, but in the case of such a configuration, the pull-up design De may be further formed continuously in the width direction. According to such a configuration, the visibility of the pull-up design De can be further improved.

**Reference Signs List**

| 1, 1A, 1B | underwear-type absorbent article |
| --- | --- |
| 5 | belt part |
| 10 | absorbent body |
| 13a | absorbent core |
| 30 | ventral waistline part |
| 40 | dorsal waistline part |
| 50, 50A | crotch elastic member |
| 115a | thread-like elastic member (leg circumference elastic member). |

**Claims**

1. An underwear-type absorbent article comprising:

   an absorbent body including a liquid-absorbent absorbent core;
   a belt part including a ventral waistline part located on one end side in a longitudinal direction of the absorbent body and a dorsal waistline part located on another end side in the longitudinal direction of the absorbent body; and
a plurality of crotch elastic members disposed on the absorbent core and extending in the longitudinal direction,
wherein at least one or more of the crotch elastic members are disposed in each of three regions divided equally in a width direction intersecting the longitudinal direction in the absorbent core.

2. The underwear-type absorbent article according to claim 1, wherein the plurality of crotch elastic members are disposed so as to straddle a center line, the center line being a bifold line crossing the longitudinal direction in the absorbent core and becoming a crotch lower end part when worn.

3. The underwear-type absorbent article according to claim 2, wherein the plurality of crotch elastic members are disposed to be biased toward a side of the ventral waistline part or a side of the dorsal waistline part with respect to the center line.

4. The underwear-type absorbent article according to claim 3, wherein the plurality of crotch elastic members are disposed to be biased toward a side where a contraction force is relatively low among the ventral waistline part and the dorsal waistline part.

5. The underwear-type absorbent article according to any one of claims 1 to 4, wherein the plurality of crotch elastic members are disposed so as not to overlap any of the ventral waistline part and the dorsal waistline part, or disposed so as to overlap one of the ventral waistline part and the dorsal waistline part and not to overlap another.

6. The underwear-type absorbent article according to claim 5, wherein

   a central part of the absorbent core in the longitudinal direction is formed in a constricted shape recessed inward in the width direction, and
   in a state where the absorbent body is unfolded, an effective length of the crotch elastic members in the longitudinal direction is shorter than a length of a part formed in the constricted shape in the longitudinal direction.

7. The underwear-type absorbent article according to any one of claims 1 to 6, wherein a distance from the crotch elastic member at an outermost end in the width direction included in the plurality of crotch elastic members to an end part in the width direction of the absorbent core is shorter than a separation distance between two of the crotch elastic members adjacent to each other included in the plurality of crotch elastic members at a center line being a bifold line crossing the longitudinal direction in the absorbent core and becoming a crotch lower end part when worn.

8. The underwear-type absorbent article according to any one of claims 1 to 7, wherein

the absorbent body includes a leg circumference elastic member stretchable in the longitudinal direction at both end parts of the absorbent body in the width direction, and
the crotch elastic members have a higher elastic modulus than the leg circumference elastic member.

9. The underwear-type absorbent article according to any one of claims 1 to 8, wherein a pull-up design indicating a pull-up point at a time of wearing is formed in at least one of a region where the absorbent body and the ventral waistline part overlap and a region where the absorbent body and the dorsal waistline part overlap.

10. The underwear-type absorbent article according to claim 9, wherein the pull-up design is formed on a line in the longitudinal direction of a maximum width part in the width direction of the absorbent core.

11. The underwear-type absorbent article according to claim 9 or 10, wherein the pull-up design is formed at an end part of the absorbent core in the longitudinal direction.

12. The underwear-type absorbent article according to any one of claims 9 to 11, wherein the pull-up design is continuously formed along the longitudinal direction from a region where the absorbent body and the ventral waistline part overlap to a region where the absorbent body and the dorsal waistline part overlap.

13. The underwear-type absorbent article according to any one of claims 9 to 12, wherein a plurality of the pull-up designs is formed apart from each other in the width direction.

14. The underwear-type absorbent article according to claim 13, wherein a pair of the pull-up designs is disposed on outer sides of the plurality of crotch elastic members so as to sandwich the plurality of crotch elastic members in the width direction.

15. The underwear-type absorbent article according to claim 14, wherein a separation distance of the pair of pull-up designs is twice or less a separation distance of the crotch elastic member at an outermost end in the width direction included in the plurality of crotch elastic members.

16. The underwear-type absorbent article according to any one of claims 1 to 15, wherein a plurality of the crotch elastic members is disposed in each of two regions divided equally in the width direction in the absorbent core.

17. An underwear-type absorbent article comprising:

an absorbent body including a liquid-absorbent absorbent core;
a belt part including a ventral waistline part located on one end side in a longitudinal direction of the absorbent body and a dorsal waistline part located on another end side in the longitudinal direction of the absorbent body; and
a plurality of crotch elastic members disposed on the absorbent core and stretchable in the longitudinal direction,
wherein the underwear-type absorbent article has a first size,
a dimension of the absorbent body when stretched is a same as a dimension of another underwear-type absorbent article of a second size, and a dimension of the belt part when stretched is a same as a dimension of the other underwear-type absorbent article, and
at least one of a number of the crotch elastic members, a contraction force, and a disposition area is larger than a number of the crotch elastic members, a contraction force, and a disposition area of the other underwear-type absorbent article.

EP 4 378 439 A1

# Fig.1

# *Fig.2*

## *Fig.3*

# *Fig.4*

1A

# *Fig.5*

## INTERNATIONAL SEARCH REPORT

| International application No. |
|---|
| **PCT/JP2022/022837** |

**A. CLASSIFICATION OF SUBJECT MATTER**

*A61F 13/49*(2006.01)i; *A61F 13/514*(2006.01)i
FI: A61F13/49 315Z; A61F13/514 400

According to International Patent Classification (IPC) or to both national classification and IPC

**B. FIELDS SEARCHED**

Minimum documentation searched (classification system followed by classification symbols)

A61F13/49; A61F13/514

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2020-049287 A (HAKUJUJI CO LTD) 02 April 2020 (2020-04-02) paragraphs [0044], [0048], [0051], [0082], [0088], fig. 7 | 17 |
| Y | | 1-16 |
| Y | JP 2011-083389 A (KAO CORP) 28 April 2011 (2011-04-28) paragraphs [0019]-[0020], fig. 1-2 | 1-16 |
| A | | 17 |
| Y | JP 2020-508146 A (THE PROCTER & GAMBLE COMPANY) 19 March 2020 (2020-03-19) paragraph [0047], fig. 4 | 9-11 |
| A | | 1-8, 12-17 |
| Y | WO 2019/221274 A1 (UNICHARM CORPORATION) 21 November 2019 (2019-11-21) paragraph [0108], fig. 8-9 | 9, 12-16 |
| A | | 1-8, 10-11, 17 |

☐ Further documents are listed in the continuation of Box C.   ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 June 2022** | **09 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

| INTERNATIONAL SEARCH REPORT | | | International application No. |
|---|---|---|---|
| Information on patent family members | | | **PCT/JP2022/022837** |

| Patent document cited in search report | | | Publication date (day/month/year) | Patent family member(s) | Publication date (day/month/year) |
|---|---|---|---|---|---|
| JP | 2020-049287 | A | 02 April 2020 | (Family: none) | |
| JP | 2011-083389 | A | 28 April 2011 | (Family: none) | |
| JP | 2020-508146 | A | 19 March 2020 | US 2019/0374403 A1 paragraph [0054], fig. 4 | |
| WO | 2019/221274 | A1 | 21 November 2019 | JP 3217272 U | |

Form PCT/ISA/210 (patent family annex) (January 2015)

**EP 4 378 439 A1**